(19) **Europäisches Patentamt**

**European Patent Office**

**Office européen des brevets**

(11) **EP 0 149 197 B2**

(12) # NEUE EUROPÄISCHE PATENTSCHRIFT

(45) Veröffentlichungstag und Bekanntmachung des Hinweises auf die Entscheidung über den Einspruch:
**08.01.1997 Patentblatt 1997/02**

(51) Int. Cl.$^6$: **A61K 9/18**, C08B 37/16

(45) Hinweis auf die Patenterteilung:
**21.03.1990 Patentblatt 1990/12**

(21) Anmeldenummer: **84115965.0**

(22) Anmeldetag: **20.12.1984**

(54) **Pharmazeutische Präparate von in Wasser schwerlöslichen oder instabilen Arzneistoffen und Verfahren zu ihrer Herstellung**

Pharmaceutical compositions containing drugs which are sparingly soluble in water or instable and methods for their preparation

Compositions pharmaceutiques contenant des médicaments peu solubles dans l'eau ou instables et procédés de leur préparation

(84) Benannte Vertragsstaaten:
**AT BE CH DE FR GB IT LI LU NL SE**

(30) Priorität: **21.12.1983 DE 3346123**

(43) Veröffentlichungstag der Anmeldung:
**24.07.1985 Patentblatt 1985/30**

(73) Patentinhaber: **JANSSEN PHARMACEUTICA N.V. 2340 Beerse (BE)**

(72) Erfinder:
• **Brauns, Ulrich D-2300 Kiel (DE)**
• **Müller, B. W. W., Prof., Dr. D-2302 Flintbek (DE)**

(74) Vertreter: **UEXKÜLL & STOLBERG Patentanwälte Beselerstrasse 4 22607 Hamburg (DE)**

(56) Entgegenhaltungen:
WO-A-82/00251    DE-A- 3 118 218
FR-A- 1 548 917    FR-A- 2 484 252
GB-A- 1 244 990    US-A- 3 453 259

• **F Fenyvesi et al. "Water soluble Cyclodextrin Polymers and their complexing properties; Proceedings of the first International Symposium on Cyclodextrin, Budapest 1981. Seiten 345-356**
• **T Cserhati et al. "Journal of Chromatography" 259 (1983). Seiten 107-110**
• **Chemical Abstracts 102:322254b**
• **NTIS order no. PB84 200 153, 250484**
• **A Harada et al. "Polymer Journal vol. 13, No.8 (1981). Seiten 777-781**
• **J Szeitly : "Cyclodextrins and their inclusion complexes", Akademiai Kiado (1982)**
• **Ullmanns Enzyklopädie der technischen Chemie, 4. Auflage, Band 18, Verlag Chemie (1979)**
• **Tetrahedron 39, 147 (1983). Seiten 1417-1474**

Bemerkungen:
Die Akte enthält technische Angaben, die nach dem Eingang der Anmeldung eingereicht wurden und die nicht in dieser Patentschrift enthalten sind.

EP 0 149 197 B2

Printed by Rank Xerox (UK) Business Services
2.13.11/3.4

**Beschreibung**

Die Erfindung betrifft pharmazeutische Präparate von in Wasser schwer löslichen oder instabilen Arzneistoffen sowie Verfahren zur deren Herstellung. Die Präparate zeichnen sich durch eine erhöhte Wasserlöslichkeit und verbesserte Stabilität aus.

Eine große Zahl von Arzneistoffen ist in Wasser nur sehr schlecht oder wenig löslich, so daß entsprechende Arzneiformen wie Tropfen oder Injektionslösungen unter Verwendung anderer polarer Hilfstoffe wie Propylenglykol usw. hergestellt werden. Falls das Arzneistoffmolekül basische oder saure Gruppen trägt, besteht ferner die Möglichkeit, durch Salzbildung die Wasserlöslichkeit zu erhöhen. In der Regel geht dies mit einer Verminderung der Wirkung oder mit einem Verlust der chemischen Stabilität einher. Aufgrund des veränderten Verteilungsgleichgewichtes vermag der Arzneistoff nämlich nur noch langsam entsprechend der Konzentration seines nichtdissoziierten Anteils in die lipophile Membran einzudringen, während der ionogene Anteil einer schnellen hydrolytischen Zersetzung unterliegen kann.

Bei der Herstellung von wässrigen Lösungen schwer wasserlöslicher Wirkstoffe verwendet man daher entweder zusätzliche "wasserähnliche" Lösungsmittel wie niedermolekulare Polyethylenglykole oder 1,2-Propylenglykol, die jedoch nicht als pharmakologisch inert zu bezeichnen sind, oder man solubilisiert den Wirkstoff unter Verwendung von Tensiden durch Einschluß der Arzneistoffmoleküle in Mizellen. Diese Solubilisation hat aber zahlreiche Nachteile: die verwendeten Tensidmoleküle wirken näufig stark hämolytisch und der Arzneistoff muß nach Applikation durch Diffusion die Mizelle verlassen. Dies führ zu einem Retardeffekt (vgl. B. W. Müller, Gelbe Reihe, Band X, S. 132 ff (1983)).

Man kann also sagen, daß es keine befriedigende und allgemein anwendbare Methode zur Lösungsvermittlung gibt.

Auch für feste Arzneistoffe ist es wichtig, daß der schwer wasserlösliche Wirkstoff in eine wasserlösliche Form gebracht wird, da eine gute Löslichkeit die Verfügbarkeit des Wirkstoffes erhöht. Es wurde schon beschrieben, daß Einschlußverbindungen z. B. mit Harnstoff oder Komplexe mit Polyvinylpyrrolidon die Löslichkeit eines Stoffes verbessern können, jedoch sind sie in wässriger Lösung nicht mehr beständig. Solche Einschlußverbindungen sind daher allenfalls für feste Arzneiformen geeignet.

Anders ist dies beim Einsatz von ($\alpha$-, $\beta$- und $\gamma$-Cyclodextrin, das einen Arzneistoff auch in wässriger Lösung in seinem Ring komplex binden kann (W. Sänger. Angew. Chemie 92, 343 (1980)). Nachteilig ist jedoch, daß das $\beta$-Cyclodextrin selbst nur schlecht wasserlösliche ist (1,8 g pro 100 ml), so daß die therapeutisch erforderlichen Arzneistoffkonzentrationen nicht erreicht werden.

Derivatitisiert man den Hilfsstoff Cyclodextrin, so kann seine Löslichkeit und damit auch die Menge des in Lösung gebrachten Arzneistoffes erheblich erhöht werden. So wird in der DE-A-31 18 218 die Lösungsvermittlung mit methyliertem $\beta$-Cyclodextrin als Monomethylderivat mit 7 Methylgruppen und insbesondere als Dimethylderivat mit 14 Methylgruppen beschrieben. Mit dem 2,6-Di-O-methylderivat kann z.B. die Löslichkeit von Indometacin in Wasser um das 20,4-fache, diejenige von Digitoxin um das 81,6-fache gesteigert werden.

E. Fenyvesi et al. (Proceedings of the first International Symposium on Cyclodextrins; Budapest 1981, Seiten 345 - 356) beschreiben die Herstellung von Cyclodextrin-Polymeren durch Reaktion mit Epichlorhydrin, die eine hohe Wasserlöslichkeit aufweisen und sich zur Erhöhung des Löslichkeit von in Wasser schwer löslichen Verbindungen eignen. Die Polymere werden ausgehend von monomerem $\beta$- und $\gamma$-Cyclodextrin hergestellt und die zur Verwendung ausgewählten Polymere besitzen ein durchschnittliches Molekulargewicht von 4000 bis 6000, mit einem mittleren Molekulargewicht $\overline{M}_W$ von 5300.

In der WO-A-820051 werden Einschlußverbindungen von Retinoiden mit Cyclodextrinen und Cyclodextrinderivaten beschrieben. Das der Veröffentlichung zugrundeliegende Problem ist die Senkung der toxischen Nebenwirkungen von Retinoiden durch Modifikation der Molekularstruktur und/oder Komplexierung mit Cyclodextrinen. Die Fettlöslichkeit der Substanzen soll verringert und damit deren Neigung zur Konzentration im Fettgewebe gesenkt oder vermieden werden. In der Veröffentlichung werden $\alpha$-, $\beta$- und $\gamma$-Cyclodextrine sowie deren Derivate für die Komplexierung als gleichermaßen geeignet bezeichnet, während als Einzelbeispiele für verwendbare Derivate in erster Linie Methoxyderivate von $\alpha$-, $\beta$- und $\gamma$-Cyclodextrinen genannt werden.

Für die therapeutische Anwendung weisen die Methylderivate des $\beta$-Cyclodextrins jedoch schwerwiegende Nachteile auf. Aufgrund ihrer erhöhten Lipophilität wirken sie hämolytisch und führen auch zu Reizungen der Schleimhäute und Augen. Ihre akute intravenöse Toxizität ist noch höher als die bereits beträchtliche Toxizität des unsubstituierten $\beta$-Cyclodextrins. Für die praktische Anwendung besteht ein weiterer schwerwiegender Nachteil darin, daß die Löslichkeit des Dimethyl-$\beta$-cyclodextrins und seiner Komplexe bei höheren Temperaturen stark abnimmt, so daß bei einem Erhitzen das Dextrin auskristallisiert. Durch diese Erscheinung wird die Sterilisation der Lösungen bei den üblichen Temperaturen von 100 bis 121°C stark erschwert.

Es wurde nun überraschenderweise gefunden, daß bestimmte andere derivatisierte $\beta$-Cyclodextrine unter Bildung von Einschlußverbindungen die Wasserlöslichkeit von schwer wasserlöslichen und die Stabilität von in Wasser instabilen Arzneistoffen ebenfalls erheblich erhöhen, die geschilderten Nachteile aber nicht aufweisen.

Gegenstand der Erfindung sind demgemäß pharmazeutische Präparate mit einem Gehalt an Einschlußverbindungen schwer wasserlöslicher oder in Wasser instabiler Arzneistoffe mit einem partiell veretherten $\beta$-Cyclodextrin, deren

Ethersubstituenten Hydroxyethyl-, Hydroxypropyl- oder Dihydroxypropylgruppen sind, wobei ein Teil der Ethersubstituenten gegebenenfalls Methyl- oder Ethylgruppen sein können und der β-Cylodextrinether eine Wasserlöslichkeit von über 1,8 g in 100 ml Wasser aufweist, wobei Einschlußverbindungen von Retinoiden mit β-Cyclodextrinethern, soweit deren Ethersubstituenten Methyl-, Ethyl- und 2-Hydroxyethylgruppen sind, sowie Einschlussverbindungen schwer wasserlöslichen oder in Wasser instabiler Arzneistoffe mit Mischungen von β-Cyclodextrin-Epichlorohydrin-Derivaten mit einem mittleren Molekulargewicht $\overline{M}_W$ von ausgenommen sind.

Die Verwendung von partiell methylierten β-Cyclodextrinethern mit 7 bis 14 Methylgruppen im β-Cyclodextrinmolekül, wie sie aus der DE-A-31 18 218 bekannt sind, fällt nicht unter die Erfindung. Ferner sind erfindungsgemäß Einschlußverbindungen von Retinoiden mit β-Cyclodextrinethern, soweit deren Ethersubstituenten Methyl-, Ethyl- und 2-Hydroxyethylgruppen sind (vgl. WO-A-820051), und Einschlussverbindungen schwer wasserlöslichen oder in Wasser instabiler Arzneistoffe mit Mischungen von β-Cyclodextrin-Epichlorohydrin-Derivaten mit einem mittleren Molekulargewicht $\overline{M}_W$ von 5300 ausgenommen.

β-Cyclodextrin ist eine ringförmig aufgebaute Verbindung aus sieben Anhydroglucoseeinheiten; sie wird auch als Cycloheptaamylose bezeichnet. Jeder der sieben Glucoseringe enthält jeweils drei Hydroxygruppen in 2-, 3- und 6-Stellung, welche verethert werden können. Bei den partiell veretherten Cyclodextrinderivaten, welche erfindungsgemäß eingesetzt werden, ist nur ein Teil dieser Hydroxygruppen mit den angegebenen Hydroxyalkylresten sowie gegebenenfalls ferner mit Methyl- oder Ethylresten verethert. Bei der Veretherung mit- 5 Hydroxyalkylresten, welche durch Umsetzung mit den entsprechenden Alkylenoxiden erfolgen kann, wird der Substitutionsgrad als molarer Substitutionsgrad (MS), nämlich in Mol Alkylenoxid je Anhydroglucoseeinheit angegeben, vgl. US-A-34 59 731, Spalte 4. Bei den erfindungsgemäß eingesetzten Hydroxyalkylethern des β-Cyclodextrins beträgt der molare Substitutionsgrad zwischen 0,2 und 10, vorzugsweise zwischen 0,2 und 2.

Besonders bevorzugt ist ein molarer Substitutionsgrad von etwa 0,25 bis etwa 1.

Für die Veretherung mit Alkylresten läßt sich unmittelbar der Substitutionsgrad (DS) je Glucoseeinheit angeben, welcher - wie bereits oben erwähnt - bei vollständiger Substitution 3 beträgt. Im Rahmen der Erfindung werden partiell veretherte β-Cyclodextrine eingesetzt, welche neben den Hydroxyalkylresten Alkylreste, nämlich Methyl- oder Ethylreste bis zu einem Substitutionsgrad von 0,2 bis 2,0, vorzugsweise 0,2 bis 1,5 enthalten. Besonders bevorzugt beträgt der Substitutionsgrad für die Alkylreste etwa 0,5 bis etwa 1,2.

Das Molverhältnis von Arzneistoff zu β-Cyclodextrinether beträgt vorzugsweise etwa 1:6 bis 4:1, insbesondere etwa 1:2 bis 1:1. In der Regel ist es bevorzugt, den Komplexbildner in einem molaren Überschuß einzusetzen.

Als Komplexbildner kommen die Hydroxyethyl-, Hydroxypropyl- und Dihydroxypropylether sowie deren entsprechende gemischte Ether und ferner Mischether mit Methyl- oder Ethylgruppen, wie z.B. Methyl-hydroxyethyl-, Methyl-hydroxypropyl-, Ethyl-hydroxyethyl- und Ethyl-hydroxypropylether des β-Cyclodextrins in Betracht.

Die Herstellung der Hydroxyalkylether des β-Cyclodextrins kann nach dem Verfahren der US-A-34 59 731 erfolgen. Geeignete Herstellungsverfahren für β-Cyclodextrinether finden sich ferner bei J. Szejtli et al., Stärke 32, 165 (1980) und A. P. Croft und R.A. Bartsch, Tetrahedron 39, 1417 (1983). Mischether des β-Cyclodextrins lassen sich herstellen, indem β-Cyclodextrin in einem ein Alkalimetallhydroxid, Wasser und ggf. mindestens ein organisches Losungsmittel (z. B. Dimethoxyethan oder Isopropanol) enthaltenden basischen flüssigen Reaktionsmedium mit mindestens zwei unterschiedlichen hydroxyalkylierenden und ggf. alkylierenden Veretherungsmitteln (z. B. Ethylenoxid, Propylenoxid, Methyl- oder Ethylchlorid) umgesetzt wird.

Arzneistoffe, die in Form von Einschlußverbindungen mit den genannten β-Cyclodextrinethern eine erheblich gesteigerte Wasserlöslichkeit bzw. eine höhere Stabilität Zeigen, sind solche, die die entsprechende Paßform haben, d.h. sie müssen in den Hohlraum des β-Cyclodextrin-Ringsystems passen. Hierzu gehören z. B. nichtsteroide Antirheumatika, Steroide, Herzglykoside und Derivate des Benzodiazepins, Benzimidazols, Piperidins, Piperazins, Imidazols oder Triazols.

Infrage kommende Benzimidazolsderivate sind Thiabendazol, Fuberidazol, Oxibendazol, Parbendazol, Carnbendazol, Mebendazol, Fenbendazol, Flubendazol, Albendazol, Oxfendazol, Nocodazol und Asternisol. Als Piperidinderivate kommen Fluspirilen, Pimozid, Penfluridol, Loperamid, Asternizol, Ketanserin, Levocabastin, Cisaprid, Altanserin, und Ritanserin in Betracht. Geeignete Piperazinderivate sind Lidoflazin, Flunarizin, Mianserin, Oxatomid, Mioflazin und Cinnarizin. Als Imidazolderivate sind Metronidazol, Ornidazol, Ipronidazol, Tinidazol, Isoconazol, Nimorazol, Burimamid, Metiamid, Metomidat, Enilconazol, Etomidat, Econazol, Clotrimazol, Carnidazol, Cimetidin, Docodazol, Sulconazol, Parconazol, Orconazol, Butoconazol, Triadiminol, Tioconazol, Valconazol, Fluotrimazol, Ketoconazol, Oxiconazol, Lombazol, Bifonazol, Oxmetidin, Fenticonazol und Tubulazol zu nennen. Zu den infrage kommenden Triazolderivaten gehören Virazol, Itraconazol und Terconazol.

Besonders wertvolle pharmazeutische Präparate werden erhalten, wenn man mit Hilfe der erfindungsgemäß verwendeten Komplexbildner Etomidat, Ketoconazol, Tubulazol, Itraconazol, Levocabastin oder Flunarizin in wasserlösliche Form überführt. Diese Präparate sind deshalb ein besonderer Gegenstand der vorliegenden Erfindung.

Die Erfindung betrifft ferner ein Verfahren zur Herstellung pharmazeutischer Zubereitungen schwer wasserlöslicher oder in Wasser instabiler Arzneistoffe, welches dadurch gekennzeichnet ist, daß man den β-Cyclodextrinether in Wasser löst und den entsprechenden Arzneistoff zufügt sowie gegebenenfalls die erhaltene Lösung der Einschlußverbindung nach an sich bekannten Methoden trocknet. Die Bildung der Lösung kann bei Temperaturen zwischen 15 und 35°C erfolgen.

Der Arzneistoff wird dabei zweckmäßigerweise portionsweise zugesetzt. Das Wasser kann beispielsweise noch physiologisch verträgliche Substanzen wie Kochsalz, Kaliumnitrat, Glukose, Mannitol, Sorbitol, Xylitol oder Puffer wie Phosphat-, Acetat- oder Citratpuffer enthalten.

Es lassen sich mit den erfindungsgemäß verwendeten β-Cyclodextrinderivaten Arzneiformen sowohl für die orale, die parenterale als auch lokale Anwendung herstellen z. B. Infusions- und Injektionslösungen, Tropfen (z. B. Augentropfen, Nasentropfen), Sprays, Aerosole, Sirups und Bäder.

Die wässrigen Lösungen können noch geeignete, physiologisch verträgliche Konservierungsmittel enthalten, z. B. quartäre Ammoniumseifen, Chlorbutanol.

Für die Herstellung fester Zubereitungen werden die Lösungen der Einschlußverbindungen nach üblichen Methoden getrocknet; z. B. kann das Wasser am Rotationsverdampfer abgezogen oder durch Gefriertrocknung entfernt werden. Der Rückstand wird pulverisiert und gegebenenfalls nach Zusatz von weiteren Hilfsstoffen in Tabletten, Dragees Suppositorien, Kapseln oder Cremes bzw. Salben überführt.

Die nachfolgenden Beispiele erläutern die Erfindung, die jedoch keinesfalls auf die hier angeführten Beispiele beschränkt ist.

Die in den Beispielen genannte Phosphatpuffer-Lösung hatte einen pH-Wert von 6.6 und folgende Zusammensetzung:

| | |
|---|---|
| $KH_2PO_4$ | 68,05 g |
| NaOH | 7,12 g |
| Aqua demin. ad | 5000,0 g |

Alle Prozentangaben sind Gewichtsprozent.

**Beispiel 1**

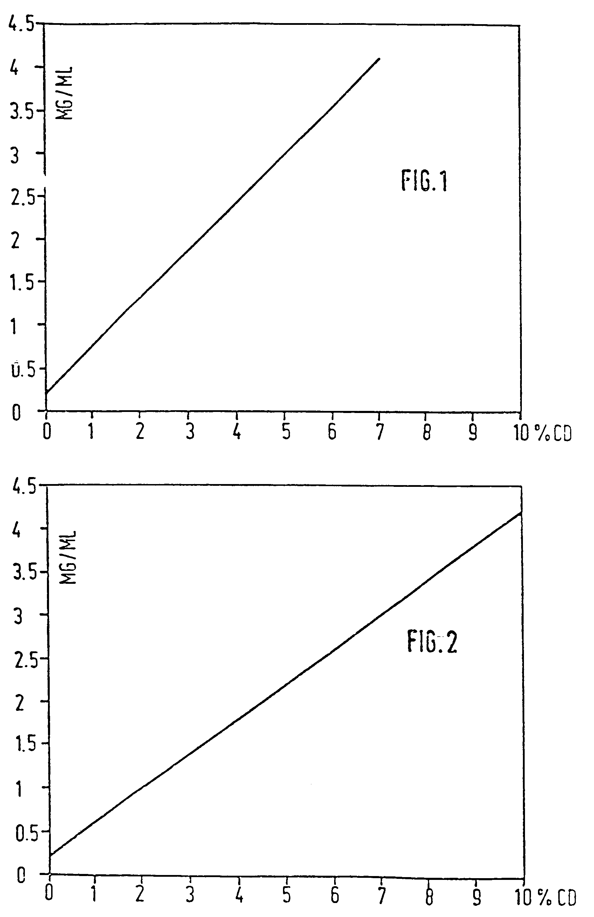

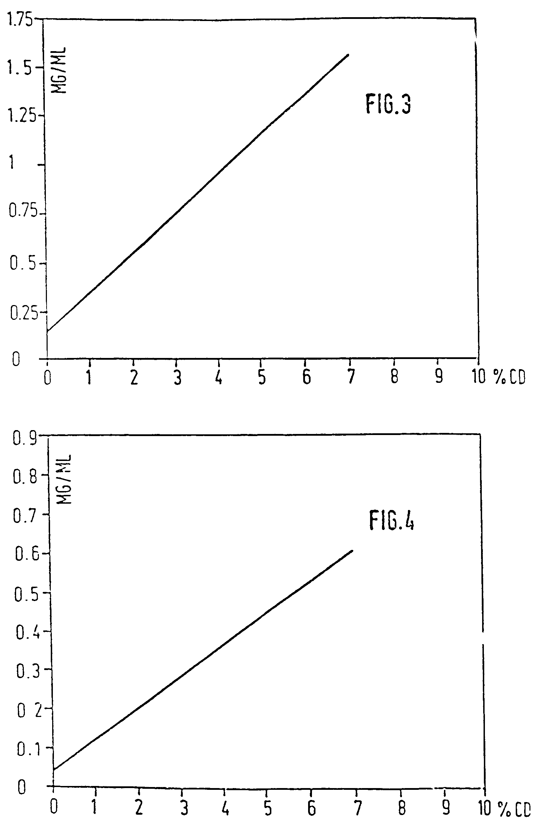

Ausgehend von einer 7 %-igen Stammlösung von Hydroxyethyl-β-cyclodextrin (MS 0.43) in Phosphatpufferlösung wurde eine Verdünnungsreihe so hergestellt, daß die Komplexbildnerkonzentration in 1 % Schritten abgestuft vorlag. 3 ml von diesen Lösungen wurden in 5 ml-Schnappdeckelgläser pipettiert, die den entsprechenden Arzneistoff enthielten. Nach 24-stündigem Schütteln bei 25°C wurde die Lösung über ein Membranfilter (0.22 μm) abfiltriert und der Gehalt an gelöstem Arzneistoff spektralphotometrisch bestimmt. Die Figuren 1, 3 und 4 zeigen die Zunahme der Arzneistoffkonzentration in Lösung in Abhängigkeit von dem Komplexbildnerzusatz für Indometacin (Fig. 1), Piroxicam (Fig. 3) und Diazepam (Fig. 4). Die maximal mögliche Arzneistoffkonzentration wird durch die Sättigungslöslichkeit des Cyclodextrinderivates im Puffer begrenzt, die im Fall des Hydroxyethyl-β-cyclodextrins (MS 0.43) bei 7,2 g/100 ml liegt.

Vergleicht man z. B. die Resultate von Indometacin mit denjenigen, die für 2,6-Di-O-methyl-β-cyclodextrin in der DE-A-3 118 218 angegeben sind (Fig. 2), so zeigt das Hydroxyethyl-Derivat eine deutlich höhere Komplexbildungskonstante (vergl. die unterschiedlichen Steigungen in Fig. 1 und 2).

**Beispiel 2**

A. Unter Verwendung einer 10 %-igen Hydroxypropyl-β-cyclodextrinlösung (MS 0.35) in Phosphatpufferlösung wurde unter denselben Bedingungen wie in Beispiel 1 die Sättigungslöslichkeit bei 25°C für verschiedene Arzneistoffe bestimmt. In Tabelle 1 sind die Sättigungslöslichkeit $S_1$ in Phosphatpufferlösung und $S_2$ in Phosphatpufferlösung unter Zusatz von 10 % Hydroxypropyl-β-cyclodextrin angegeben.

Tabelle 1

| Arzneistoff | $S_1$ (mg/ml) | $S_2$ (mg/ml) | Verh.$S_1$ zu $S_2$ | |
|---|---|---|---|---|
| Indometacin | 0,19 | 5,72 | 1: | 30,1 |
| Digitoxin | 0,002 | 1,685 | 1: | 842,5 |
| Progesteron | 0,0071 | 7,69 | 1: | 1083,0 |
| Dexamethason | 0,083 | 14,28 | 1: | 172,0 |
| Hydrocortison | 0,36 | 21,58 | 1: | 59,9 |
| Diazepam | 0,032 | 0,94 | 1: | 29,4 |

B. In ähnlicher Weise wurde die Löslichkeit von Arzneistoffen in einer 4 %-igen wässrigen Lösung von Hydroxypropyl-methyl-β-cyclodextrin (DS = 0,96; MS = 0,43) untersucht. Die erhaltenen Ergebnisse sind in der nachfolgenden Tabelle 2 zusammengefaßt, wobei für jeden Arzneistoff das Verhältnis R von Sättigungslöslichkeit in Wasser bzw. beim angegebenen pH-Wert mit und ohne Zusatz des β-Cyclodextrinderivats angegeben ist. In allen Fällen erwiesen sich die erfindungsgemäß hergestellten Lösungen darüber hinaus im Vergleich zu wässrigen Lösungen als wesentlich stabiler.

Tabelle 2

| Arzneistoff | | R |
|---|---|---|
| Itraconazol | bei pH 5 | 96 |
| | bei pH 2,5 | 75 |
| Flunarizin | | 18 |
| Levocabastin | bei pH 9,5 | 81 |
| | bei pH 7,4 | 8 |
| Ketoconazol | | 85 |
| Flubendazol | | 30 |
| Tubulazol | | 43 |
| Cisaprid | | 3 |
| Loperamid | | 62 |
| Etomidat | | 8,5 |
| Cinnarizin | bei pH 5 | 28 |
| | bei pH 3 | 12 |

**Beispiel 3**

In 10 ml Phosphatpufferlösung wurden bei 25°C 0,7 g Hydroxyethyl-β-cyclodextrin (MS 0.43) und 0,04 g Indometacin bis zur klaren Lösung eingearbeitet. Diese wurde durch ein Membranfilter (0,22 µm) filtriert und unter Laminarflow in eine vorsterilisierte Injektionsflasche abgefüllt, die bei 21°C eingelagert wurde (B). Parallel dazu wurde eine gesättigte Indometaeinlösung in Phosphatpufferlösung (0,21 mg/ml) unter gleichen Bedingungen aufbewahrt (A). Die hochdruckflüssigkeitschromatographisch ermittelten Wirkstoff-Konzentrationen sind in Tabelle 3 wiedergegeben. Die wesentlich verbesserte Stabilität des erfindungsgemäßen Präparates ist offensichtlich.

Tabelle 3

| Lagerzeit in Wochen | Gehalt an Indometacin (%) | |
|---|---|---|
| | A | B |
| 0 | 100,1 | 99,7 |
| 2 | 91,2 | 99,9 |
| 4 | 79,1 | 98,1 |
| 6 | 69,8 | 98,6 |
| 8 | 64,8 | 98,4 |

**Beispiel 4** (Injektionspräparat)

Man löste 0,35 g Hydroxypropyl-β-cyclodextrin (MS 0.35) in 5 ml physiologischer Kochsalzlösung, temperierte auf ca. 35°C und gab 3 mg Diazepam hinzu. Nach kurzem Rühren erhielt man eine klare Lösung, die nach Filtration durch ein 0,45 μm Membranfilter in eine Ampulle abgefüllt wurde.

**Beispiel 5** (Tablette)

In 100 ml Wasser wurden 7 g Hydroxyethyl-β-cyclodextrin (MS 0.43) sowie 0,5 g Medroxyprogesteronacetat gelöst. Anschließend wurde das Wasser auf einem Rotationsverdampfer abgezogen. Der Rückstand (75 mg) wurde pulverisiert und mit 366 mg Calcium-hydrogenphosphat. $2H_2O$, 60 mg Maisstärke, 120 mg Cellulosepulver(mikrokristallin), 4,2 mg hochdisperse Kieselsäure (Aerosil® 200) und 4,8 mg Magnesiumstearat zu Tabletten mit einem Endgewicht von 630,0 mg und pro Einzeldosis 5 mg Wirkstoff verarbeitet. Die Lösungsgeschwindigkeit des Medroxyprogesteronacetats aus dieser Zubereitung ist etwa 21 mal höher als bei Einsatz der gleichen Tablettenhilfsstoffe ohne Zusatz des β-Cyclodextrinethers.

**Beispiel 6**

In 100 ml Wasser oder Zuckerlösung (5 %-ige wässrige Lösung) wurden 5 g Hydroxyethyl-β-cyclodextrin (MS 0.43) und 14 mg Vitamin A-acetat durch Rühren über ca. 2,5 h unter Stickstoffatmosphäre gelöst. Nach Filtration über ein 0,45 μm Membranfilter wurde die Lösung entweder in Ampullen abgefüllt und sterilisiert oder unter Verwendung von 0,4 % Chlorbutanol als Konservierungsmittel in Tropfflaschen abgefüllt.

**Beispiel 7**

In 100 ml physiologischer Kochsalzlösung wurden 5 g bzw. 7,5 g Hydroxyethyl-β-cyclodextrin (MS 0.43) und 0,5 bzw. 0,75 g Lidocain bei 30°C gelöst (B). Analog Beispiel 6 wurden daraus Injektionslösungen, Augentropfen und Lösungen für die cutane Anwendung hergestellt. Vergleicht man die anaethesierende Wirkung dieser Losungen am Tiermodell gegenüber einer wässrigen Lidocain-HCI-Lösung (A), so ist eine Wirkungsverlängerung von 300 % festzustellen. Versuch: Ratte, Injektion von 0,1 ml in die Schwanzwurzel in die Nähe der rechten oder linken Nervenstränge und elektrische Reizung. Die Versuchsergebnisse sind in Tabelle 4 zusammengefaßt.

Tabelle 4

| Arzneistoffkonz. (%) | Wirkungsdauer (min) | | Verlängerung (%) |
|---|---|---|---|
| | A | B | |
| 0,5 | 56 | 163 | 291 |
| 0,75 | 118 | 390 | 330 |

**Beispiel 8**

Es wurden 6 mg Dexamethason und 100 mg Hydroxyethyl-β-cyclodextrin (MS 0.43) in 5 ml Wasser gelöst, über ein

0,22 μm Membranfilter steril filtriert und in eine Dosier-Aerosoldose verpackt, die 0,1 ml pro Dosis freizusetzen erlaubt.

**Beispiel 9**

Die akute intravenöse Toxizität von einigen β-Cyclodextrinen wurde an Ratten untersucht, wobei die nachfolgenden Ergebnisse erhalten wurden. Dabei zeigte sich, daß die Toxizität der erfindungsgemäß eingesetzten Derivate überraschend eine ganze Größenordnung niedriger ist.

Tabelle 5

| $LD_{50}$ bei Ratten (i.v.) in mg/kg Körpergewicht | |
|---|---|
| β-Cyclodextrin | 453 |
| Dimethyl-β-cyclodextrin (DS = 2,0) | 200 - 207 |
| Hydroxypropyl-methyl-β-cyclodextrin (DS = 0,96; MS = 0,43) | > 2000* |

\* eine höhere Dosis wurde nicht getestet. Bei der Maus lag der Wert > 4000 mg/kg.

In einer weiteren Versuchsreihe wurde die hämolytische Wirkung der Methylether gemäß DE-A-3 118 218 mit derjenigen eines erfindungsgemäß verwendeten Ethers verglichen. Dazu wurden 100 μl einer physiologischen Kochsalzlösung mit einem Cyclodextringehalt von 10 %, 800 μl eines Puffers (400 mg MOPS, 36 mg $Na_2HPO_4 \cdot 2 H_2O$, 1,6 g NaCl in 200 ml $H_2O$) und 100 μl einer Suspension von roten Blutkörperchen (menschlichen Ursprungs, dreimal mit Kochsalzlösung gewaschen), 30 Min. lang bei 37°C gemischt. Anschließend wurde zentrifugiert und die Extinktion bei 540 mn bestimmt.
Kontrollen:

a) 100 μl Kochsalzlösung + Puffer → 0 % Hämolyse
b) 900 μl Wasser → 100 % Hämolyse

Die gefundenen Ergebnisse sind in der nachfolgenden Tabelle 6 zusammengefaßt, wobei die Konzentrationen angegeben sind, bei denen eine 50 %-ige bzw. eine 100 % Hämolyse stattfand.

Tabelle 6

| Substanz | $C_{50}$ % | $C_{100}$ % |
|---|---|---|
| Dimethyl-β-CD (DS = 2.0) | 0,33 % | 0,5 % |
| Methyl-β-CD (DS = 1,79) | 0,53 % | 0,8 % |
| Hydroxypropyl-methyl-β-CD (DS = 0,96 MS = 0,43 %) | 1,5 % | 4 % |

Die Ergebnisse zeigen, daß die hämolytische Wirkung des Hydroxypropyl-methylethers etwa 5 bis 8-mal geringer ist als die des Dimethylethers gemäß Stand der Technik.
Tierversuche haben ferner ergeben, daß die Hydroxyalkylether im Gegensatz zu den Methylethern keine Irritation der Schleimhäute und Augen hervorrufen.

**Patentansprüche**

1.  Pharmazeutisches Präparat mit einem Gehalt an Einschlußverbindungen schwer wasserlöslicher oder in Wasser instabiler Arzneistoffe mit einem Cyclodextrin, dadurch gekennzeichnet, daß das Cyclodextrin ein partiell verethertes β-Cyclodextrin ist, dessen Ethersubstituenten Hydroxyethyl-, Hydroxypropyl- oder Dihydroxypropylgruppen sind und ein Teil der Ethersubstituenten gegebenenfalls Methyl- oder Ethylgruppen sein können und der β-Cyclodextrinether eine Wasserlöslichkeit von über 1,8 g in 100 ml Wasser aufweist, wobei Einschlußverbindungen von Retinoiden mit β-Cyclodextrinen soweit deren Ethersubstituenten Methyl-, Ethyl- oder 2-Hydroxyethylgruppen sind, sowie Einschlußverbindungen schwer wasserlöslicher oder in Wasser instabiler Arzneistoffe mit Mischungen von β-Cyclodextrin-Epichlorhydrin-Derivaten mit einem mittleren Molekulargewicht $\overline{M}_W$ von 5300 ausgenommen sind.

2. Präparat nach Anspruch 1, dadurch gekennzeichnet, daß das partiell veretherte β-Cyclodextrin einen molaren Substitutionsgrad für die Hydroxyalkylreste von 0,2 bis 10 und einen Substitutionsgrad für die Alkylreste von 0,2 bis 2,0 aufweist.

3. Präparat gemäß einem der Ansprüche 1 oder 2, dadurch gekennzeichnet, daß es Arzneistoff und β-Cyclodextrinether im Molverhältnis von 1:6 bis 4:1 enthält.

4. Präparat gemäß einem der Ansprüche 1 bis 3, dadurch gekennzeichnet, daß es als Arzneistoff ein nicht steroides Antirheumatikum, ein Steroid, ein Herzglycosid oder Derivate des Benzodiazepins, Benzimidazols, Piperidins, Piperazins, Imidazols oder Triazols enthält.

5. Präparat nach einem der Ansprüche 1 bis 4, dadurch gekennzeichnet, daß es als Arzneistoff Etomidat enthält.

6. Präparat nach einem der Ansprüche 1 bis 4, dadurch gekennzeichnet, daß es als Arzneistoff Ketoconazol enthält.

7. Präparat nach einem der Ansprüche 1 bis 4, dadurch gekennzeichnet, daß es als Arzneistoff Itraconazol enthält.

8. Präparat nach einem der Ansprüche 1 bis 4, dadurch gekennzeichnet, daß es als Arzneistoff Levobastin enthält.

9. Präparat nach einem der Ansprüche 1 bis 4, dadurch gekennzeichnet, daß es als Arzneistoff Flunarizin enthält.

10. Präparat nach einem der Ansprüche 1 bis 4, dadurch gekennzeichnet, daß es als Arzneistoff Tubulazol enthält.

11. Verfahren zur Herstellung eines pharmazeutischen Präparates gemäß einem der Ansprüche 1 bis 10, dadurch gekennzeichnet, daß man den β-Cyclodextrinether in Wasser löst und den entsprechenden Arzneistoff zufügt sowie ggf. die erhaltene Lösung der Einschlußverbindung nach an sich bekannten Methoden trocknet.

12. Verfahren nach Anspruch 11, dadurch gekennzeichnet, daß man den nach Entfernen des Lösungsmittels erhaltenen Rückstand pulverisiert und, gegebenenfalls nach Zusatz weiterer Hilfsstoffe, in eine feste Applikationsform überführt.

13. Verfahren nach einem der Ansprüche 11 oder 12, dadurch gekennzeichnet, daß man dem Wasser noch weitere physiologisch verträgliche Stoffe zusetzt.

14. Verfahren nach Anspruch 13, dadurch gekennzeichnet, daß man dem Wasser Kochsalz, Glucose, Mannitol, Sorbitol, Xylitol oder einen Phosphat- oder Citratpuffer zusetzt.

## Claims

1. A pharmaceutical preparation containing inclusion complexes of medicinal substances which are sparingly water-soluble or are instable in water, with a cyclodextrin, characterized in that the cyclodextrin is a partially etherified β-cyclodextrin, the ether substituents of which are hydroxyethyl, hydroxypropyl or dihydroxypropyl groups, wherein one portion of the ether substituents may optionally be methyl or ethyl groups and the β-cyclodextrin ether has a water-solubility of more than 1.8 g in 100 ml of water, wherein inclusion complexes of retinoids with β-cyclodextrin ethers insofar as their ether substituents are methyl, ethyl and 2-hydroxyethyl groups, as well as inclusion complexes of medicinal substances which are sparingly water-soluble or are instable in water, with mixtures of β-cyclodextrin epichlorohydrin derivatives having a mean molecular weight ($\overline{M}_W$) of 5300 are excluded.

2. A preparation according to Claim 1, characterized in that it contains a partially etherified β-cyclodextrin which has a molar degree of substitution for the hydroxyalkyl substituents of from 0.2 to 10 and a degree of substitution for the alkyl substituents of from 0.2 to 2.0.

3. A preparation according to either Claim 1 or Claim 2, characterized in that it contains the medicinal substance and the β-cyclodextrin ether in a molar ratio of from 1:6 to 4:1.

4. A preparation according to any one of Claim 1 to 3, characterized in that as medicinal substance it contains a non-steroid antirheumatic agent, a steroid, a cardiac glycoside or derivatives of benzodiazepin, benzimidazole, piperidine, piperazine, imidazole or triazole.

5. A preparation according to any one of Claims 1 to 4, characterized in that as medicinal substance it contains etomidate.

6. A preparation according to any one of Claims 1 to 4, characterized in that as medicinal substance it contains ketoconazole.

7. A preparation according to any one of Claims 1 to 4, characterized in that as medicinal substance it contains itraconazole.

8. A preparation according to any one of Claims 1 to 4, characterized in that as medicinal substance it contains levocabastine.

9. A preparation according to any one of Claims 1 to 4, characterized in that as medicinal substance it contains flunarizine.

10. A preparation according to any one of Claims 1 to 4, characterized in that as medicinal substance it contains tubulazole.

11. A process for the preparation of a pharmaceutical preparation according to any one of Claims 1 to 10, characterized in that the $\beta$-cyclodextrin ether is dissolved in water and the corresponding medicinal substance is added and, optionally, the resultant solution of the inclusion complex is dried in accordance with per se known methods.

12. A process according to Claim 11, characterized in that the residue obtained after removal of the solvent is pulverised and, optionally after the addition of further adjuvants, is converted into a solid administration form.

13. A process according to Claim 11 or Claim 12, characterized in that further physiologically compatible substances are added to the water.

14. A process according to Claim 13, characterized in that common salt, glucose, mannitol, sorbitol, xylitol or a phosphate or citrate buffer is added to the water.

**Revendications**

1. Composition pharmaceutique contenant des composés d'inclusion de médicaments peu solubles dans l'eau ou instables dans l'eau et d'une cyclodextrine, caractérisée par le lait que la cyclodextrine est une bêta-cyclodextrine partiellement éthérifiée dont les radicaux d'éthers sont des groupes hydroxyéthyle, hydroxypropyle ou dihydroxypropyle et une partie des radicaux d'éthers peut consister le cas échéant en groupes méthyle ou éthyle et l'éther de bêta-cyclodextrine ayant une solubilité dans l'eau supérieure à 1,8 g pour 100 ml d'eau, sous réserve que les composés d'inclusion des rétinoïdes et des bêta-cyclodextrine sont exclus lorsque leurs radicaux d'éthers sont des groupes méthyle, éthyle et 2-hydroxyéthyle, de même que des composés d'inclusion peu solubles dans l'eau ou instables dans l'eau et les mélanges de dérivés de bêta-cyclodextrlne-épichlorhydrine avec un poids moléculaire moyen ($\overline{M}_w$) de 5300 sont exclus.

2. Composition selon la revendication 1, caractérisée en ce que la bêta-cyclodextrine partiellement éthérifiée présente un degré de substitution molaire de 0,2 à 10 pour les groupes hydroxyalkyle et un degré de substitution de 0,2 à 2,0 pour les groupes alkyle.

3. Composition selon l'une des revendications 1 ou 2, caractérisée en ce qu'elle contient le médicament et l'éther de bêta-cyclodextrine dans un rapport molaire de 1:6 à 4:1.

4. Composition selon l'une des revendications 1 à 3, caractérisée en ce qu'elle contient en tant que médicament un médicament antirhumatismal non stéroïdique, un stéroïde, un cardioglucoside ou des dérivés de la benzodiazépine, du benzimidazole, de la pipéridine, de la pipérazine, de l'imidazole ou du triazole.

5. Composition selon l'une des revendications 1 à 4, caractérisée en ce qu'elle contient en tant que médicament l'Etomidat.

**6.** Composition selon l'une des revendications 1 à 4, caractérisée en ce qu'elle contient en tant que médicament le Ketoconazol.

**7.** Composition selon l'une des revendications 1 à 4, caractérisée en ce qu'elle contient en tant que médicament l'Itraconazol.

**8.** Composition selon l'une des revendications 1 à 4, caractérisée en ce qu'elle contient en tant que médicament le Levocabastin.

**9.** Composition selon l'une des revendications 1 à 4, caractérisée en ce qu'elle contient en tant que médicament le Flunarizin.

**10.** Composition selon l'une des revendications 1 à 4, caractérisée en ce qu'elle contient en tant que médicament le Tubulazol.

**11.** Procédé de préparation d'une composition pharmaceutique selon l'une des revendications 1 à 10, caractérisé en ce que l'on dissout l'éther de bêta-cyclodextrine dans l'eau et on ajoute le médicament voulu, puis, le cas échéant, on sèche la solution du composé d'inclusion ainsi obtenue par des procédés connus en soi.

**12.** Procédé selon la revendication 11, caractérisé en ce que l'on broie le résidu obtenu après élimination du solvant et, le cas échéant, après addition d'autres produits auxiliares, on met sous une forme d'administration solide.

**13.** Procédé selon l'une des revendications 11 ou 12, caractérisé en ce que l'on ajoute encore à l'eau d'autres substances acceptables pour l'usage pharmaceutique.

**14.** Procédé selon la revendication 13, caractérisé en ce que l'on ajoute à l'eau du chlorure de sodium, du glucose, du mannitol, du sorbitol, du xylitol ou un tampon au phosphate ou au citrate.

FIG.1

FIG.2

FIG.3

FIG.4